# EUROPÄISCHE PATENTSCHRIFT

(11) **EP 4 464 686 B1**
(45) Veröffentlichungstag und Bekanntmachung des Hinweises auf die Patenterteilung: **16.07.2025**
(21) Anmeldenummer: 23173654.7
(22) Anmeldetag: 16.05.2023
(51) Int. Cl.: C07C 41/54, C07C 43/303, C07D 317/12

(54) **VERFAHREN ZUR HERSTELLUNG EINES ACETALS AUS EINEM OLEFIN UNTER EINSATZ VON PTI2 ODER PTBR2**
PROCESS FOR THE PREPARATION OF AN ACETAL FROM AN OLEFIN USING TTI2 OR PTBR2
PROCÉDÉ DE PRÉPARATION D'UN ACÉTAL À PARTIR D'UNE OLÉFINE À L'AIDE DE PTI2 OU DE PTBR2

(43) Veröffentlichungstag der Anmeldung: 20.11.2024
(73) Patentinhaber: Evonik Oxeno GmbH & Co. KG, 45772 Marl (DE)
(72) Erfinder: FRANKE, Robert, 45772 Marl (DE); SCHNEIDER, Carolin, 40789 Monheim am Rhein (DE); JACKSTELL, Ralf, 18106 Rostock (DE); BELLER, Matthias, 18211 Ostseebad Nienhagen (DE)
(74) Vertreter: Evonik Patent Association

(56) Entgegenhaltungen:
- WO-A1-95/06025
- US-A- 5 688 972
- OLIVIER DIEBOLT ET AL: "Formation of Acetals under Rhodium-Catalyzed Hydroformylation Conditions in Alcohols", ADVANCED SYNTHESIS AND CATALYSIS, JOHN WILEY & SONS, INC, HOBOKEN, USA, vol. 354, no. 4, 23 February 2012 (2012-02-23), pages 670 - 677, XP072357599, ISSN: 1615-4150, DOI: 10.1002/ADSC.201100707
- PETOCZ G ET AL: "Xantphos as cis- and trans-chelating ligand in square-planar platinum(II) complexes. Hydroformylation of styrene with platinum-xantphos-tin(II)chloride system", JOURNAL OF ORGANOMETALLIC CHEMISTRY, ELSEVIER, AMSTERDAM, NL, vol. 689, no. 7, 1 April 2004 (2004-04-01), pages 1188 - 1193, XP004496421, ISSN: 0022-328X, DOI: 10.1016/J.JORGANCHEM.2003.10.045

## Beschreibung

Die vorliegende Erfindung betrifft ein Verfahren zur Herstellung eines Acetals aus einem Olefin unter Einsatz von PtI₂ oder PtBr₂.

WO9506025A1 beschreibt ein Verfahren zur Herstellung eines Acetals durch Hydroformylierung einer ethylenisch ungesättigten organischen Verbindung. Ein Artikel von Olivier Diebolt et al. in Adv. Synth. Catal. 2012, 354, 670-677 beschreibt die Bildung von Acetalen unter den Bedingungen der Rhodium-katalysierten Hydroformylierung in Alkoholen. Ein Artikel von György Petöcz et al. in Journal of Organometallic Chemistry 689 (2004) 1188-1193 beschreibt eine Hydroformylierung von Styrol mit einem Platin-Xantphos-Zinn(II)chlorid System.

Der vorliegenden Erfindung lag die Aufgabe zugrunde, ein Verfahren zur Herstellung eines Acetals aus einem Olefin bereitzustellen. Hierbei soll eine gute Ausbeute erzielt werden.

Diese Aufgabe wird gelöst durch ein Verfahren gemäß Anspruch 1.

Verfahren umfassend die Verfahrensschritte:
a) Vorlegen eines Olefins;
b) Zugabe einer Verbindung gemäß der Formel (I): 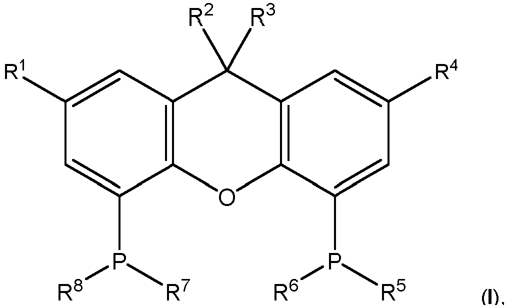
   wobei R¹, R², R³, R⁴, R⁵, R⁶, R⁷, R⁸ ausgewählt sind aus: -H, -(C₁-C₁₂)-Alkyl, -(C₆-C₂₀)-Aryl;
   und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) Zugabe von Ptl_{z} oder PtBr₂;
d) Zugabe eines Alkohols, welcher ausgewählt ist aus: Methanol, Ethanol, 1-Propanol, 1-Butanol, 1-Pentanol, 1-Hexanol, 1-Heptanol, 1-Oktanol, Ethan-1,2-diol, 1,2-Propandiol, 1,3-Propandiol, 1,4-Butandiol;
e) Zuführen von CO und H₂;
f) Erwärmen des Reaktionsgemisches aus a) bis e), wobei das Olefin zu einem Acetal umgesetzt wird.

Hierbei können die Verfahrensschritte a) bis e) in beliebiger Reihenfolge erfolgen. Üblicherweise erfolgt die Zugabe von CO und H₂ jedoch, nachdem die Reaktionspartner in den Schritten a) bis d) vorgelegt wurden.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Variante des Verfahrens sind R², R³, R⁵, R⁶, R⁷, R⁸ ausgewählt aus: -(C₁-C₁₂)-Alkyl, -(C₆-C₂₀)-Aryl.

In einer Variante des Verfahrens stehen R⁵, R⁶, R⁷, R⁸ für -(C₆-C₂₀)-Aryl.

In einer Variante des Verfahrens stehen R⁵, R⁶, R⁷, R⁸ für -Ph

In einer Variante des Verfahrens stehen R² und R³ für -(C₁-C₁₂)-Alkyl.

In einer Variante des Verfahrens stehen R² und R³ für -CH₃.

In einer Variante des Verfahrens stehen R¹ und R⁴ für -H.

In einer Variante des Verfahrens weisen die Verbindung (I) die Struktur (1) auf:

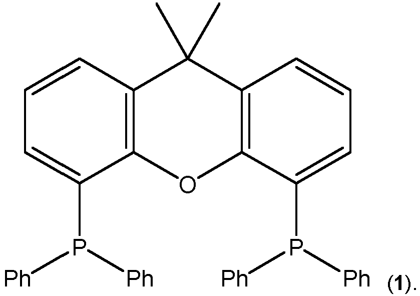

In einer Variante des Verfahrens wird in Verfahrensschritt c) PtI₂ zugegeben.

In einer Variante des Verfahrens wird in Verfahrensschritt c) PtBr₂ zugegeben.

In einer Variante des Verfahrens ist der Alkohol in Verfahrensschritt d) ausgewählt aus: Methanol, Ethanol, 1-Propanol, 1-Butanol, Ethan-1,2-diol, 1,2-Propandiol.

In einer Variante des Verfahrens ist der Alkohol in Verfahrensschritt d) MeOH.

In einer Variante des Verfahrens erfolgt das Zuführen von CO und H₂ bei einem Druck in einem Bereich von 1 MPa (10 bar) bis 6 MPa (60 bar).

In einer Variante des Verfahrens erfolgt das Zuführen von CO und H₂ bei einem Druck in einem Bereich von 3 MPa (30 bar) bis 5 MPa (50 bar).

In einer Variante des Verfahrens ist das Olefin ausgewählt aus: Ethen, Propen, 1-Buten, cis- und/oder trans-2-Buten, iso-Buten, 1,3-Butadien, 1,2-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, Tri-n-Buten, 1,7-Octadien, 1,9-Decadien, Methyl-9-decenoat (9-Dame) oder Mischungen davon. oder Mischungen davon.

In einer Variante des Verfahrens weist das Olefin zwei Doppelbindungen auf.

In einer Variante des Verfahrens weist das Olefin zwei endständige Doppelbindungen auf.

In einer Variante des Verfahrens ist das Olefin 1,3-Butadien.

In einer Variante des Verfahrens erfolgt das Erwärmen auf eine Temperatur im Bereich von 50 °C bis 150°C.

In einer Variante des Verfahrens erfolgt das Erwärmen auf eine Temperatur im Bereich von 80 °C bis 140°C.

In einer Variante des Verfahrens umfasst dieses den zusätzlichen Verfahrensschritt e'):
e') Zugabe eines Lösungsmittels, welches kein Alkohol ist.

In einer Variante des Verfahrens ist das Lösungsmittel ausgewählt aus: THF, MTBE, DCM, ACN, Heptan, DMF, Toluol, Xylen, Mesitylen, Dibenzyltoluol.

Im Folgenden soll die Erfindung anhand von Ausführungsbeispielen näher erläutert werden.

### Allgemeine Versuchsbeschreibung

In einen Edelstahl Autoklav der Firma Parr Instruments werden unter Argon das Olefin, ein inertes Lösungsmittel, der Alkohol, PtX₂ (X = Halogen) und der Ligand platziert. Es wird Synthesegas CO/H₂ (1:1) aufgepresst und die Reaktion bei der gewählten Reaktionstemperatur unter Rührung durchgeführt. Nach dem Ende der Reaktionszeit wird der Autoklav auf Raumtemperatur heruntergekühlt, der Restdruck abgelassen und eine GC-Probe zu Bestimmung der Ausbeute an Zielprodukt entnommen und gemessen.

### Variation des Alkohols 1-Octen

### Reaktionsbedingungen:

10 mmol 1-Octen, 0,5 mol% Ptl₂, 2,2 äq. Xantphos (1), Lösungsmittel: Toluol, p(CO/H₂): 40 bar, T: 80 °C, t: 24 h.
Monoalkohol: 4 Äquivalente, Diol: 2 Äquivalente

| Acetal | Ausbeute [%] |
|---|---|
| 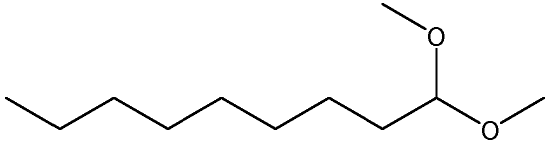 | 90 |
| 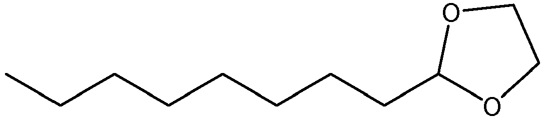 | 48 |
| 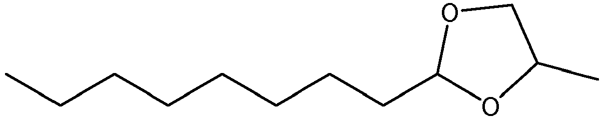 | 75 |
| 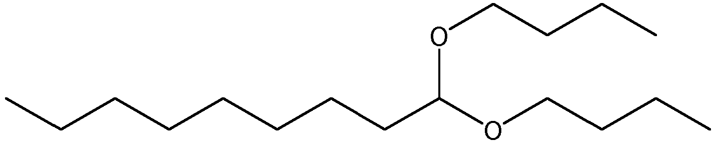 | 81 |

### Variation des Alkohols 1,3-Butadien

### Reaktionsbedingungen:

10 mmol 1,3-Butadien, 0,5 mol% Ptl₂, 2,2 äq. Xantphos (1), Lösungsmittel: Toluol, p(CO/H₂): 40 bar, T: 80 °C, t: 24 h.
Monoalkohol: 4 Äquivalente, Diol: 2 Äquivalente

| Acetal | Ausbeut e [%] |
|---|---|
| 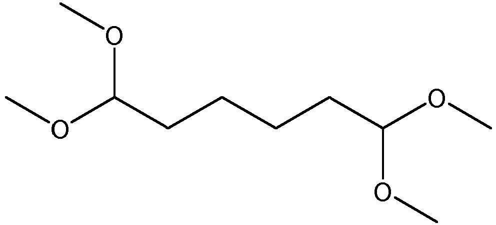 | 55 |
| 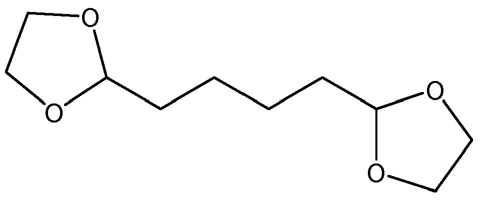 | 86 |
| 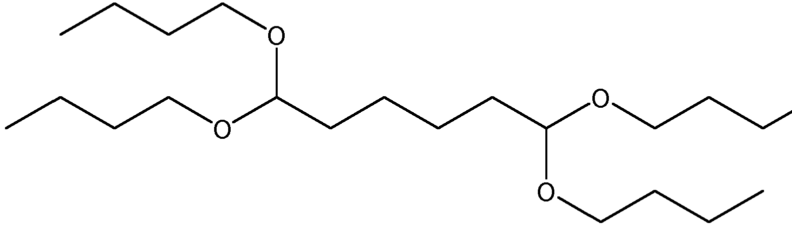 | 50 |

### Variation des Halogens

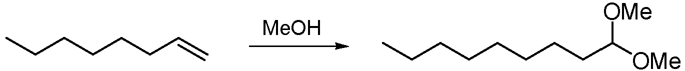

10 mmol 1-Octen, 40 mmol Methanol, 10 ml Toluol, PtX₂ (0,1 mol %), Xantphos (1) (0,22 mol %) werden in einen 25 ml Autoklaven unter Argon gegeben. Es werden 40 bar Synthesegas (CO/H₂ = 1.1) aufgepresst. Die Reaktion wird bei 120 °C unter Rührung und unter Messung des Gasverbrauchs im Autoklaven (elektronischer Druckaufnehmer, Specview Software) 24 h durchgeführt. Der Autoklav wird auf Raumtemperatur heruntergekühlt und der Druck wird abgelassen. Zur Bestimmung der Ausbeute wird eine GC-Probe genommen.

Die Reaktion wird für X = I / Br / CI durchgeführt.

### Reaktionsbedingungen:

10 mmol 1-Octen, 0,1 mol% PtX₂, 2,2 äq. Xantphos (1), Lösungsmittel: Toluol, p(CO/H₂): 40 bar, T: 120 °C, t: 24 h.

### Ausbeuten:

Ptl₂: 46 %
PtBr₂: 36 %
PtCl₂*: 7 %
* nicht erfindungsgemäßer Vergleichsversuch

Wie die Versuchsergebnisse zeigen, wird die Aufgabe durch das erfindungsgemäße Verfahren gelöst.

## Patentansprüche

1. Verfahren umfassend die Verfahrensschritte:
a) Vorlegen eines Olefins;
b) Zugabe einer Verbindung gemäß der Formel (I): 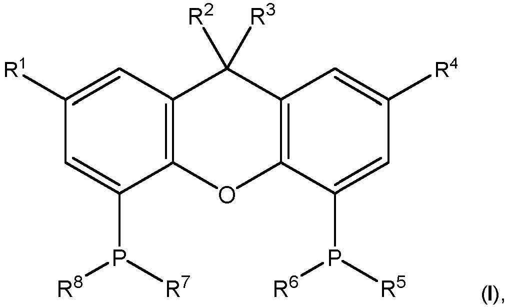
wobei R¹, R², R³, R⁴, R⁵, R⁶, R⁷, R⁸ ausgewählt sind aus: -H, -(C₁-C₁₂)-Alkyl, -(C₆-C₂₀)-Aryl;
und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) Zugabe von Ptl_{z} oder PtBr₂;
d) Zugabe eines Alkohols, welcher ausgewählt ist aus: Methanol, Ethanol, 1-Propanol, 1-Butanol, 1-Pentanol, 1-Hexanol, 1-Heptanol, 1-Oktanol, Ethan-1,2-diol, 1,2-Propandiol, 1,3-Propandiol, 1,4-Butandiol;
e) Zuführen von CO und H₂;
f) Erwärmen des Reaktionsgemisches aus a) bis e), wobei das Olefin zu einem Acetal umgesetzt wird.

2. Verfahren nach Anspruch 1,
wobei R², R³, R⁵, R⁶, R⁷, R⁸ ausgewählt sind aus: -(C₁-C₁₂)-Alkyl, -(C₆-C₂₀)-Aryl.

3. Verfahren nach einem der Ansprüche 1 oder 2,
wobei R⁵, R⁶, R⁷, R⁸ für -(C₆-C₂₀)-Aryl stehen.

4. Verfahren nach einem der Ansprüche 1 bis 3,
wobei R² und R³ für -(C₁-C₁₂)-Alkyl stehen.

5. Verfahren nach einem der Ansprüche 1 bis 4,
wobei R¹ und R⁴ für -H stehen.

6. Verfahren nach einem der Ansprüche 1 bis 5,
wobei die Verbindung **(I)** die Struktur **(1)** aufweist:

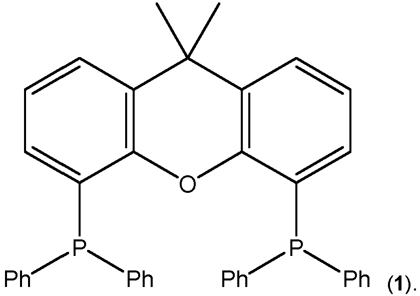

7. Verfahren nach einem der Ansprüche 1 bis 6,
wobei in Verfahrensschritt c) Ptl_{z} zugegeben wird.

8. Verfahren nach einem der Ansprüche 1 bis 6,
wobei in Verfahrensschritt c) PtBr₂ zugegeben wird.

9. Verfahren nach einem der Ansprüche 1 bis 8,
wobei der Alkohol in Verfahrensschritt d) ausgewählt ist aus: Methanol, Ethanol, 1-Propanol, 1-Butanol, Ethan-1,2-diol, 1,2-Propandiol.

10. Verfahren nach einem der Ansprüche 1 bis 9,
wobei der Alkohol in Verfahrensschritt e) MeOH ist.

11. Verfahren nach einem der Ansprüche 1 bis 10,
wobei das Zuführen von CO und H₂ bei einem Druck erfolgt in einem Bereich von 1 MPa (10 bar) bis 6 MPa (60 bar).

12. Verfahren nach einem der Ansprüche 1 bis 11,
wobei das Zuführen von CO und H₂ bei einem Druck erfolgt in einem Bereich von 3 MPa (30 bar) bis 5 MPa (50 bar).

13. Verfahren nach einem der Ansprüche 1 bis 12,
wobei das Olefin ausgewählt ist aus: Ethen, Propen, 1-Buten, cis- und/oder trans-2-Buten, iso-Buten, 1,3-Butadien, 1,2-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, Tri-n-Buten, 1,7-Octadien, 1,9-Decadien, Methyl-9-decenoat (9-Dame) oder Mischungen davon.

14. Verfahren nach einem der Ansprüche 1 bis 13,
wobei das Olefin zwei Doppelbindungen aufweist.

15. Verfahren nach einem der Ansprüche 1 bis 13,
wobei das Olefin zwei endständige Doppelbindungen aufweist.

## Claims

1. Process comprising the process steps of:
a) initially charging an olefin;
b) adding a compound of formula (I): 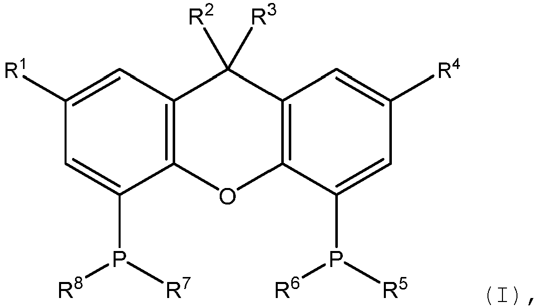
where R¹, R², R³, R⁴, R⁵, R⁶, R⁷, R⁸ are selected from: -H, - (C₁-C₁₂) -alkyl, - (C₆-C₂₀) -aryl;
and, if R¹, R², R³, R⁴, R⁵, R⁶, R⁷, R⁸ are -(C₆-C₂₀)-aryl, the aryl ring may have substituents selected from: -(C₁-C₁₂) -alkyl, -O- (C₁-C₁₂) -alkyl;
c) adding PtI₂ or PtBr₂;
d) adding an alcohol selected from: methanol, ethanol, 1-propanol, 1-butanol, 1-pentanol, 1-hexanol, 1-heptanol, 1-octanol, ethane-1,2-diol, propane-1,2-diol, propane-1,3-diol, butane-1,4-diol;
e) feeding in CO and H₂;
f) heating the reaction mixture from a) to e), to convert the olefin to an acetal.

2. Process according to Claim 1,
where R², R³, R⁵, R⁶, R⁷, R⁸ are selected from: -(C₁-C₁₂)-alkyl, -(C₆-C₂₀)-aryl.

3. Process according to either of Claims 1 and 2, where R⁵, R⁶, R⁷, R⁸ are -(C₆-C₂₀)-aryl.

4. Process according to any of Claims 1 to 3,
where R² and R³ are -(C₁-C₁₂)-alkyl.

5. Process according to any of Claims 1 to 4,
where R¹ and R⁴ are -H.

6. Process according to any of Claims 1 to 5,
wherein the compound (I) has the structure (1):

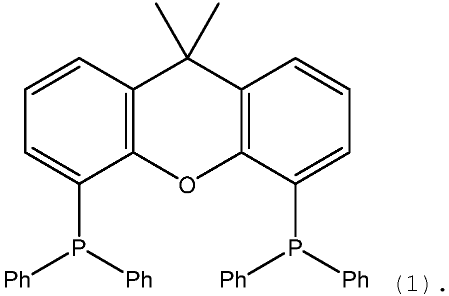

7. Process according to any of Claims 1 to 6,
wherein PtI₂ is added in process step c).

8. Process according to any of Claims 1 to 6,
wherein PtBr₂ is added in process step c).

9. Process according to any of Claims 1 to 8,
wherein the alcohol in process step d) is selected from: methanol, ethanol, 1-propanol, 1-butanol, ethane-1,2-diol, propane-1,2-diol.

10. Process according to any of Claims 1 to 9,
wherein the alcohol in process step e) is MeOH.

11. Process according to any of Claims 1 to 10,
wherein CO and H₂ are fed in at a pressure in a range from 1 MPa (10 bar) to 6 MPa (60 bar).

12. Process according to any of Claims 1 to 11,
wherein CO and H₂ are fed in at a pressure in a range from 3 MPa (30 bar) to 5 MPa (50 bar).

13. Process according to any of Claims 1 to 12,
wherein the olefin is selected from: ethene, propene, 1-butene, cis- and/or trans-2-butene, isobutene, 1,3-butadiene, 1,2-butadiene, 1-pentene, cis- and/or trans-2-pentene, 2-methyl-1-butene, 3-methyl-1-butene, 2-methyl-2-butene, hexene, tetramethylethylene, heptene, 1-octene, 2-octene, di-n-butene, tri-n-butene, 1,7-octadiene, 1,9-decadiene, methyl 9-decenoate (9-Dame) or mixtures thereof.

14. Process according to any of Claims 1 to 13,
wherein the olefin has two double bonds.

15. Process according to any of Claims 1 to 13,
wherein the olefin has two terminal double bonds.

## Revendications

1. Procédé comprenant les étapes de procédé :
a) disposition préalable d'une oléfine ;
b) ajout d'un composé selon la formule (I) : 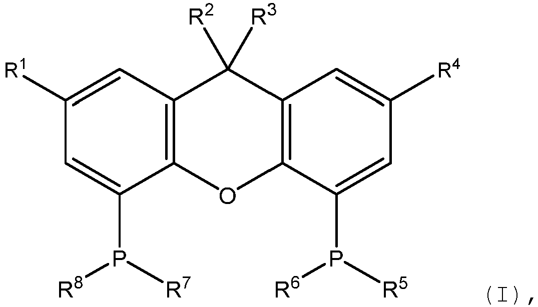
R¹, R², R³, R⁴, R⁵, R⁶, R⁷, R⁸ étant choisis parmi : -H, (C₁-C₁₂) -alkyle, (C₆-C₂₀) -aryle ;
et pour le cas où R¹, R², R³, R⁴, R⁵, R⁶, R⁷, R⁸ représentent - (C₆-C₂₀) -aryle, le cycle aryle pouvant présenter des substituants qui sont choisis parmi : -(C₁-C₁₂)-alkyle, - O- (C₁-C₁₂) -alkyle ;
c) ajout de PtI₂ ou de PtBr₂ ;
d) ajout d'un alcool qui est choisi parmi : le méthanol, l'éthanol, le 1-propanol, le 1-butanol, le 1-pentanol, le 1-hexanol, le 1-heptanol, le 1-octanol, l'éthane-1,2-diol, le 1,2-propanediol, le 1,3-propanediol, le 1,4-butanediol ;
e) introduction de CO et de H₂ ;
f) chauffage du mélange réactionnel provenant de a) à e), l'oléfine étant convertie en acétal.

2. Procédé selon la revendication 1,
R², R³, R⁵, R⁶, R⁷, R⁸ étant choisis parmi : - (C₁-C₁₂) - alkyle, - (C₆-C₂₀) -aryle.

3. Procédé selon l'une des revendications 1 ou 2,
R⁵, R⁶, R⁷, R⁸ représentant (C₆-C₂₀) -aryle.

4. Procédé selon l'une des revendications 1 à 3,
R² et R³ représentant - (C₁-C₁₂) -alkyle.

5. Procédé selon l'une des revendications 1 à 4,
R¹ et R⁴ représentant -H.

6. Procédé selon l'une des revendications 1 à 5,
le composé (I) présentant la structure (1) :

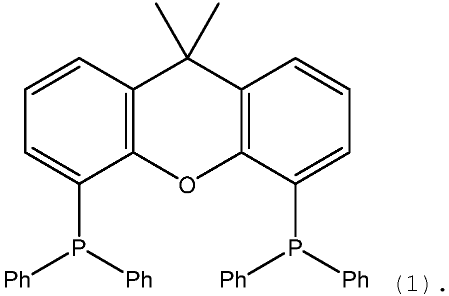

7. Procédé selon l'une des revendications 1 à 6,
du PtI₂ étant ajouté dans l'étape de procédé c).

8. Procédé selon l'une des revendications 1 à 6,
du PtBr₂ étant ajouté dans l'étape de procédé c).

9. Procédé selon l'une des revendications 1 à 8, l'alcool dans l'étape de procédé d) étant choisi parmi : le méthanol, l'éthanol, le 1-propanol, le 1-Butanol, l'éthane-1,2-diol, le 1,2-propanediol.

10. Procédé selon l'une des revendications 1 à 9, l'alcool dans l'étape de procédé e) étant MeOH.

11. Procédé selon l'une des revendications 1 à 10, l'introduction de CO et de H₂ ayant lieu sous une pression dans la plage de 1 MPa (10 bars) à 6 MPa (60 bars).

12. Procédé selon l'une des revendications 1 à 11, l'introduction de CO et de H₂ ayant lieu sous une pression dans la plage de 3 MPa (30 bars) à 5 MPa (50 bars).

13. Procédé selon l'une des revendications 1 à 12, l'oléfine étant choisie parmi : l'éthylène, le propène, le 1-butène, le cis-2-butène et/ou le trans-2-butène, l'isobutène, le 1,3-butadiène, le 1,2-butadiène, le 1-pentène, le cis-2-pentène et/ou le trans-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène, le tri-n-butène, le 1,7-octadène, le 1,9-décadiène, le 9-décénoate de méthyle (9-Dame) ou leurs mélanges.

14. Procédé selon l'une des revendications 1 à 13, l'oléfine présentant deux doubles liaisons.

15. Procédé selon l'une des revendications 1 à 13, l'oléfine présentant deux doubles liaisons en position d'extrémité.
